Europäisches Patentamt

(19) European Patent Office

Office européen des brevets

(11) Publication number: **0 183 249**
**B1**

(12) **EUROPEAN PATENT SPECIFICATION**

(45) Date of publication of patent specification: 01.03.89

(51) Int. Cl.⁴: **C 07 C 120/00,**
C 07 C 121/66, C 07 C 121/48

(21) Application number: 85115072.2

(22) Date of filing: 27.11.85

(54) Process for preparing alpha-arylacrylonitriles.

(30) Priority: 29.11.84 US 676479
17.05.85 US 735151
17.05.85 US 735148
17.05.85 US 735146

(43) Date of publication of application:
04.06.86 Bulletin 86/23

(45) Publication of the grant of the patent:
01.03.89 Bulletin 89/09

(84) Designated Contracting States:
AT BE CH DE FR GB IT LI LU NL SE

(56) References cited:
DE-B-1 087 122
US-A-2 864 851
US-A-4 132 728

PATENT ABSTRACTS OF JAPAN, vol. 5, no. 140
(C-70)812r, 4th September 1981; & JP - A - 56
75467 (MITSUBISHI KASEI KOGYO) 22-06-1981

(73) Proprietor: ETHYL CORPORATION
Ethyl Tower 451 Florida Boulevard
Baton Rouge Louisiana 70801 (US)

(72) Inventor: Ramachandran, Venkataraman
1446 Brookhollow Drive
Baton Rouge Louisiana 70810 (US)

Inventor: Maloney, John Robert
1257 Briarridge Drive
Baton Rouge Louisiana 70810 (US)

Inventor: Davidson, Robert Ira
13156 Briargrove Avenue
Baton Rouge Louisiana 70810 (US)

(74) Representative: Dipl.-Ing. Schwabe, Dr. Dr.
Sandmair, Dr. Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to alpha-arylacrylonitriles and more particularly to a process for preparing them.

It is known that alpha-arylacrylonitriles are useful as chemical intermediates and that they can be prepared in various ways. For example, Jacobs et al., *Journal of Organic Chemistry*, 1983, Vol. 48, pp. 5134—5135, teach that 6-methoxy-1-cyano-3,4-dihydro-naphthalene is useful as an intermediate in the synthesis of steroids and that it can be prepared by (1) the addition of diethylaluminum cyanide to 6-methoxytetralone followed by dehydration or (2) the addition of cyanotrimethylsilane to 6-methoxytetralone followed by treatment with phosphoryl chloride in pyridine. As taught by Jacobs et al., the former method of synthesizing their alpha-arylacrylonitrile is impractical for large scale operations, and the latter method requires two steps.

An object of this invention is to provide a novel process for preparing alpha-arylacrylonitriles.

Another object is to provide such a process which is suitable for large scale operations and produces the alpha-arylacrylonitriles from aryl ketones in a single step.

These and other objects are attained by reacting an aryl ketone having a removable hydrogen alpha to the carbonyl group with hydrogen cyanide, a tri- or tetraalkylammonium cyanide or a metal cyanide with a Lewis acid.

Aryl ketones that can be used in the practice of the invention have the formula Ar—CO—R wherein Ar is aryl and R is a monovalent aliphatic, cycloaliphatic, or aromatic group having a removable hydrogen in the alpha-position. In such ketones the Ar group is generally an aryl group containing 6—20 carbons, most commonly a phenyl or naphthyl group which optionally bears one or more inert substituents, i.e., substituents that do not inhibit the activity of the Lewis acid in removing the removable hydrogen, such as alkyl, alkylthio, alkoxy, halo or nitro. The R group is generally a saturated or unsaturated aliphatic, cycloaliphatic, or aromatic group containing 1—20 carbons optionally bearing one or more inert substituents and sometimes joined with the Ar group to form a fused ring.

Exemplary of such ketones are phenyl alkyl ketones wherein the alkyl group is methyl, ethyl, propyl, isopropyl, butyl, isobutyl, t-butyl, pentyl, hexyl, heptyl, octyl, nonyl, decyl or dodecyl; the corresponding substituted-phenyl alkyl ketones wherein the substituents on the benzene ring may be any of the aforementioned alkyl groups and/or the corresponding alkoxy or alkylthio groups, chloro, bromo or nitro; the corresponding naphthyl or substituted-naphthyl alkyl ketones; the corresponding aryl substituted-alkyl ketones wherein the substituents on the alkyl group may be any of aforementioned inert substituents; the corresponding aryl substituted-or-unsubstituted-cycloalkyl (e.g., cyclopropyl, cyclobutyl, cyclohexyl or cyclooctyl) ketones; the corresponding aryl substituted-or-unsubstituted-alkenyl ketones wherein the unsaturation is at least one carbon removed from the carbon bearing the removable hydrogen, such as ketones in which the alkenyl group is 2-butenyl, 3-hexenyl, 4-hexenyl or 4-octenyl; the corresponding aryl substituted-or-unsubstituted-cycloalkenyl ketones; the corresponding aryl substituted-or-unsubstituted-aromatic ketones wherein said aromatic group is benzyl, phenylethyl or phenylpropyl; tetralone. Among the preferred ketones are acetophenones, such as acetophenone, 4-chloroacetophenone, 4-isobutylacetophenone or 4-ethoxyacetophenone and tetralones, such as tetralone, 6-methoxytetralone or 7-bromotetralone.

The Lewis acid utilized in the reaction may be any suitable Lewis acid, generally hydrogen fluoride, a trialkylaluminum, or, more preferably, a metal halide, such as boron or aluminum trifluoride, triiodide, trichloride, or tribromide, tin tetrachloride, zinc dichloride, gallium trichloride, titanium tetrachloride, diethylaluminum chloride, ethylaluminum dichloride, ethoxyaluminum dichloride, diethoxyaluminum chloride, hydroxyaluminum dichloride, dihydroxyaluminum chloride, and other such compounds wherein at least one halogen is attached to a metal atom, any remaining valences of which are usually satisfied by hydroxy, hydrocarbyl, or hydrocarbyloxy groups, generally hydroxy or alkyl or alkoxy groups containing 1—10 carbons. The preferred Lewis acids are boron trifluoride and aluminum chloride, especially aluminum chloride. This ingredient of the reaction mixture is ordinarily employed in the amount of 0.5—1.5, preferably 1—1.1, mols per mol of aryl ketone, although smaller or larger amounts can be employed if desired.

The cyanide ion that is reacted with the aryl ketone is provided by hydrogen cyanide, a tri- or tetraalkylammonium cyanide (generally such a compound containing up to 50 carbons) such as trimethylammonium cyanide, tributylmethylammonium cyanide or tetrabutylammonium cyanide, or a metal cyanide, such as cuprous cyanide or an alkali or alkaline earth metal cyanide such as lithium, sodium, potassium, rubidium, cesium, beryllium, magnesium, calcium, strontium, or barium cyanide. The sodium, potassium, and hydrogen cyanides are generally the preferred sources of cyanide ion. The amount of cyanide ion employed is not critical, but it is usually desirable to employ 1—5, preferably 1—2, mols of cyanide ion per mol of aryl ketone to produce good yields of product.

Other ingredients that are suitably included in the reaction mixture are a solvent and a phase transfer catalyst. Solvents that may be employed include all solvents in which the reactants are soluble, such as aliphatic and aromatic hydrocarbons (e.g., toluene, xylenes or heptanes), chlorobenzene or nitrobenzene; but the preferred solvent is generally nitrobenzene. Particularly useful phase transfer catalysts are tetraalkylammonium halides (generally such halides containing up to about 50 carbons), preferably bromides and chlorides, such as tetrabutyl-ammonium bromide or tributylmethylammonium chloride. When employed, the catalyst is used in a catalytic amount, e.g., 2—6% by weight of the aryl ketone; and its use sometimes seems to permit the attainment of higher yields than can be obtained in its absence.

In the practice of the invention, the ingredients of the reaction mixture may be combined in any suitable manner, preferably with the solids in finely-divided form, and heated at a suitable temperature, e.g., 60—120°C., preferably 70—90C., to produce the desired product. Lower temperatures can be used but are less desirable because of their leading to slower reactions; higher temperatures are apt to be undesirable because of the tendency for by-products to be formed at the higher temperatures. The time required to obtain good yields varies with the temperature but is frequently in the range of 4—10 hours.

It is sometimes preferred to combine the ingredients by prestirring the cyanide ion source, the Lewis acid, and a solvent before combining these ingredients with the aryl ketone, and it appears to be desirable to maintain the temperature of these ingredients below 60°C., e.g., at 10—50°C., conveniently at 20—30°C., until the addition of the aryl ketone has been completed.

It is also sometimes preferred to conduct the cyanation in the presence of a small amount of water and/or concentrated HCl — additives which appear to effect an activation of one or more of the reactants and increase yields. The particular amount of water and/or HCl employed is an activating amount, i.e., an amount insufficient to hydrolyze the Lewis acid completely, and may be provided simply by the water naturally present in one or more of the aforementioned ingredients of the reaction mixture. When it is desired to employ additional water and/or HCl, the added amount is generally in the range of 0.1—1.0 mol per mol of the aryl ketone.

After completion of the reaction, the product can be recovered by conventional means or, alternatively, can be subjected to further reactions without being isolated when the further reactions would not be inhibited by impurities in the crude product. It is frequently desirable to subject the alpha-arylacrylonitrile to subsequent reactions. One such reaction is a dehydrogenation of a product such as 6-methoxy-1-cyano-3,4-dihydro-naphthalene to a product such as 6-methoxy-1-cyanonaphthalene — a dehydrogenation that can be accomplished, e.g., by heating the reaction mixture, preferably at reflux temperatures, in the presence of a palladium-on-carbon catalyst or by other techniques known in the art.

The invention is particularly advantageous as a one-step, commercially-acceptable process for preparing alpha-arylacrylonitriles, especially 1-cyano-3,4-dihydronaphthalenes, that can then be converted to other products.

## Example I

A mixture of 1.3 g of dry AlCl$_3$, 0.64 g of dry NaCN, and 87 mg of tetrabutylammonium bromide (TBAB) in 8.7 ml of dry nitrobenzene (NB) was stirred for two hours under a nitrogen atmosphere. Then 1.53 g of 6-methoxytetralone (6-MT) were added to provide a reaction mixture containing the 6-MT, NaCN and AlCl$_3$ in a mol ratio of 1/1.5/1.1 and containing 5.6% of TBAB, based on the weight of 6-MT. The reaction mixture was stirred at 90°C. for 10 hours to form 6-methoxy-1-cyano-3,4-dihydronaphthalene (6-MCDN). After workup the VPC ratio of 6-MT/6-MCDN was determined to be 8/92. The process resulted in an 85% isolated yield of 6-MCDN.

## Example II

Example I was essentially repeated except that the AlCl$_3$/NaCN/TBAB/NB mixture was not subjected to the two hour stirring period prior to the addition of the 6-MT. After workup the VPC ratio of 6-MT/6-MCDN was determined to be 41/59.

## Example III

Example I was essentially repeated except that the 6-MT was replaced with 4-methoxyphenyl 3-chloropropyl ketone and the amount of NaCN was reduced to only 1.3 molar proportions. VPC analysis showed a 70% conversion of the ketone to alpha-(4-methoxyphenyl)-beta-(2-chloroethyl)-acrylonitrile.

## Example IV

A mixture of 1.56 g of boron trifluoride etherate 0.98 g of NaCN, and 100 mg of TBAB in 10 ml of NB was stirred for two hours. Then 1.76 g of 6-MT were added to provide a reaction mixture containing the 6-MT, NaCN, and boron trifluoride in a mol ratio of 1/2/1.1. The mixture was heated at 90°C. for two hours and then at 120°C. for six hours to form 6-MCDN. Analysis showed the 6-MT/6-MCDN ratio to be 5/4.

## Example V

A solution of 22.7 g of anhydrous AlCl in 100 ml of NB was cooled to 10°C. in an ice bath, after which 6.9 g of liquid HCN were added. The mixture was stirred vigorously and 30 g of 6-MT were added to provide a reaction mixture containing the 6-MT, HCN, and AlCl$_3$ in a mol ratio of 1/1.5/1. When the 6-MT had completely dissolved, the mixture was transferred to an autoclave and heated at 70°C. for ten hours. After cooling, the contents of the autoclave were removed and treated with 100 ml of dilute HCl and 100 ml of methylene chloride. The mixture was shaken in a separatory funnel and allowed to stand for phase separation. The lower organic layer was removed and concentrated on a rotary evaporator to remove methylene chloride. GC investigation (internal standard method) of the NB solution showed an 88% yield of 6-MCDN.

### Example VI

An 8.47 g portion of $AlCl_3$ was added under nitrogen in a dry box to a suitable reaction vessel, followed by the addition of a 50 ml portion of dry nitrobenzene. The resulting mixture was stirred for 15 minutes, after which 5.57 g of powdered NaCN and 0.50 g of dry tetrabutylammonium bromide (TBAB) were successively added. The resulting yellow slurry was stirred for two hours. Then 10 g of distilled 6-methoxytetralone (6-MT) were added to provide a green slurry containing the 6-MT, NaCN, $AlCl_3$, and TBAB in a mol ratio of 1/2/1.1/0.03, and the reaction mixture was heated to 90°C. and maintained at that temperature for six hours. Analysis of the slurry after completion of the reaction showed it to contain 78.8% 6-MT and 19.5% 6-methoxy-1-cyano-3,4-dihydronaphthalene (6-MCDN) by GC area %.

### Example VII

Example VI was essentially repeated except that 0.25 g of concentrated HCl was added to the reaction mixture after the addition of the TBAB had been completed, and the reaction mixture was maintained at 90°C. for only four hours. Analysis of the final reaction mixture showed it to contain 7 area % 6-MT and 89.4 area % 6-MCDN.

### Example VIII

Example VI was essentially repeated except that three drops of water were added to the reaction mixture after the addition of the TBAB had been completed. Analysis of the final reaction mixture showed it to contain 4.4 area % 6-MT and 90.5 area % 6-MCDN.

## Claims

1. A process for preparing alpha-arylacrylonitriles wherein an aryl ketone corresponding to the formula Ar—CO—R, wherein Ar is aryl, R is a monovalent aliphatic, cycloaliphatic or aromatic group having a removable hydrogen in the alpha-position, is reacted with hydrogen cyanide, a tri- or tetraalkylammonium cyanide or a metal cyanide as cyanide ion source and a Lewis acid to form an arylacrylonitrile corresponding to the formula Ar—C(CN)=R' wherein Ar is aryl and R' is the divalent group obtained by removing the removable hydrogen from R.

2. The process of claim 1 wherein the aryl ketone is an acetophenone.

3. The process of claim 2 wherein the acetophenone is 4-isobutylacetophenone.

4. The process of claim 1 wherein the aryl ketone is a tetralone.

5. The process of claim 4 wherein the tetralone is 6-methoxytetralone.

6. The process of claim 1 wherein the cyanide ion source is hydrogen cyanide.

7. The process of claim 1 wherein the cyanide ion source is a metal cyanide.

8. The process of claim 7 wherein the cyanide ion source is an alkali metal cyanide.

9. The process of claim 8 wherein the alkali metal cyanide is sodium cyanide.

10. The process of claim 8 wherein the alkali metal cyanide is potassium cyanide.

11. The process of claim 1 wherein the Lewis acid is a metal halide.

12. The process of claim 11 wherein the metal halide is aluminum chloride.

13. The process of claim 1 wherein the reaction is conducted in the presence of a catalytic amount of a phase transfer catalyst.

14. The process of claim 13 wherein the catalyst is a tetraalkylammonium halide.

15. The process of claim 14 wherein the tetraalkylammonium halide is tetrabutylammoniumbromide.

16. The process of claim 1 wherein the reaction is conducted in a solvent.

17. The process of claim 16 wherein the solvent is nitrobenzene.

18. The process of claim 1 wherein the reaction is conducted in the presence of a small amount of water and/or HCl.

19. The process of claim 18 wherein the reaction is conducted in the presence of 0.1—1.0 mol of added water and/or HCl per mol of aryl ketone.

20. The process of claim 1 wherein the reaction is conducted at a temperature of 60 to 120°C.

21. A process for preparing a 1-cyano-3,4-dihydronaphthalene which comprises reacting a tetralone having a removable hydrogen alpha to the carbonyl group with an alkali metal cyanide and aluminum chloride in the presence of a catalytic amount of a tetraalkylammonium halide at a temperature of 60—120°C.

22. The process of claim 21 wherein the tetralone is 6-methoxytetralone and the product is 6-methoxy-1-cyano-3,4-dihydronaphthalene.

## Patentansprüche

1. Verfahren zur Herstellung von alpha-Arylacrylnitrilen, bei dem ein Arylketon entsprechend der Formel Ar—CO—R, worin Ar Aryl bedeutet, R eine monovalente aliphatische, cycloaliphatische oder aromatische Gruppe mit einem in alpha-Position ablösbaren Wasserstoff ist, umgesetzt wird mit Wasserstoffcyanid, einem Tri- oder Tetraalkylammoniumcyanid oder einem Metallcyanid und einer Lewis-Säure unter Bildung eines Arylacrylnitrils entsprechend der Formel Ar—C(CN)=R', worin Ar Aryl bedeutet und R' die divalente Gruppe ist, die durch Entfernen des ablösbaren Wasserstoffs von R erhalten wurde.

2. Verfahren nach Anspruch 1, bei dem das Arylketon ein Acetophenon ist.

3. Verfahren nach Anspruch 2, bei dem das Acetophenon 4-Isobutylacetophenon ist.

4. Verfahren nach Anspruch 1, bei dem das Arylketon ein Tetralon ist.

5. Verfahren nach Anspruch 4, bei dem das Tetralon 6-Methoxytetralon ist.

6. Verfahren nach Anspruch 1, bei dem die Cyanidionenquelle ein Wasserstoffcyanid ist.

7. Verfahren nach Anspruch 1, bei dem die Cyanidionenquelle ein Metallcyanid ist.

8. Verfahren nach Anspruch 7, bei dem die Cyanidionenquelle ein Alkalimetallcyanid ist.

9. Verfahren nach Anspruch 8, bei dem das Alkalimetallcyanid Natriumcyanid ist.

10. Verfahren nach Anspruch 8, bei dem das Alkalimetallcyanid Kaliumcyanid ist.

11. Verfahren nach Anspruch 1, bei dem die Lewis-Säure ein Metallhalogenid ist.

12. Verfahren nach Anspruch 11, bei dem das Metallhalogenid Aluminiumchlorid ist.

13. Verfahren nach Anspruch 1, bei dem die Reaktion in Gegenwart einer katalytischen Menge eines Phasentransferkatalysators durchgeführt wird.

14. Verfahren nach Anspruch 13, bei dem der Katalysator ein Tetraalkylammoniumhalogenid ist.

15. Verfahren nach Anspruch 14, bei dem das Tetraalkylammoniumhalogenid Tetrabutylammoniumbromid ist.

16. Verfahren nach Anspruch 1, bei dem die Reaktion in einem Lösungsmittel durchgeführt wird.

17. Verfahren nach Anspruch 16, bei dem das Lösungsmittel Nitrobenzol ist.

18. Verfahren nach Anspruch 1, bei dem die Reaktion in Gegenwart einer geringen Menge Wasser und/oder HCl durchgeführt wird.

19. Verfahren nach Anspruch 18, bei dem die Reaktion in Gegenwart von 0,1—1,0 Mol zugesetzten Wassers und/oder HCl pro Mol Arylketon durchgeführt wird.

20. Verfahren nach Anspruch 1, bei dem die Reaktion bei einer Temperatur von 60 bis 120°C durchgeführt wird.

21. Verfahren zur Herstellung eines 1-Cyano-3,4-dihydronaphthalins, bei dem ein Tetralon mit einem ablösbaren alpha-Wasserstoff zur Carbonylgruppe mit einem Alkalimetallcyanid und Aluminiumchlorid in Gegenwart einer katalytischen Menge von Tetraalkylammoniumhalogenid bei einer Temperatur von 60—120°C umgesetzt wird.

22. Verfahren nach Anspruch 21, bei dem das Tetralon das Produkt 6-Methoxytetralon und das Produkt 6-Methoxy-1-cyano-3,4-dihydronaphthalin ist.

## Revendications

1. Procédé de préparation d'alpha-arylacrylonitriles, dans lequel une arylcétone correspondant à la formule Ar—CO—R, dans laquelle Ar représente un groupe aryle, R représente un groupe aliphatique, cycloaliphatique ou aromatique monovalent ayant en position alpha un atome d'hydrogène pouvant être éliminé, est amenée à réagir avec l'acide cyanhydrique, uyn cyanure de tri- ou tétra-alkylammonium ou un cyanure métallique, servant de source d'ions cyanure, et un acide de Lewis pour former un arylacrylonitrile correspondant à la formule Ar—C(CN)=R' dans laquelle Ar représente un groupe aryle et R' représente le groupe divalent obtenu par élimination de R de l'atome d'hydrogène pouvant être éliminé.

2. Procédé suivant la revendication 1, dans lequel l'arylcétone est une acétophénone.

3. Procédé suivant la revendication 2, dans lequel l'acétophénone est la 4-isobutylacétophénone.

4. Procédé suivant la revendication 1, dans lequel l'arylcétone est une tétralone.

5. Procédé suivant la revendication 4, dans lequel la tétralone est la 6-méthoxytétralone.

6. Procédé suivant la revendication 1, dans lequel la source d'ion cyanure est l'acide cyanhydrique.

7. Procédé suivant la revendication 1, dans lequel la source d'ion cyanure est un cyanure métallique.

8. Procédé suivant la revendication 7, dans lequel la source d'ion cyanure est un cyanure de métal alcalin.

9. Procédé suivant la revendication 8, dans lequel le cyanure de métal alcalin est le cyanure de sodium.

10. Procédé suivant la revendication 8, dans lequel lè cyanure de métal alcalin est le cyanure de potassium.

11. Procédé suivant la revendication 1, dans lequel l'acide de Lewis est un halogénure métallique.

12. Procédé suivant la revendication 11, dans lequel l'halogénure métallique est le chlorure d'aluminium.

13. Procédé suivant la revendication 1, dans lequel la réaction est conduite en présence d'une quantité catalytique d'un catalyseur de transfert de phase.

14. Procédé suivant la revendication 13, dans lequel le catalyseur est un halogénure de tétra-alkylammonium.

15. Procédé suivant la revendication 14, dans lequel l'halogénure de tétra-alkylammonium est le bromure de tétrabutylammonium.

16. Procédé suivant la revendication 1, dans lequel la réaction est conduite dans un solvant.

17. Procédé suivant la revendication 16, dans lequel le solvant est le nitrobenzène.

18. Procédé suivant la revendication 1, dans lequel la réaction est conduite en présence d'une petite quantité d'eau et/ou de HCl.

19. Procédé suivant la revendication 18, dans lequel la réaction est conduite en présence de 0,1 à 1,0 mole d'eau ajoutée et/ou de HCl par mole d'arylcétone.

20. Procédé suivant la revendication 1, dans lequel la réaction est conduite à une température de 60 à 120°C.

21. Procédé de préparation d'un 1-cyano-3,4-dihydronaphtalène, qui consiste à faire réagir une tétralone ayant en position alpha du groupe carbonyle un atome d'hydrogène pouvant être éliminé, avec un cyanure de métal alcalin et le chlorure d'aluminium en présence d'une quantité catalytique d'un halogénure de tétra-alkylammonium à une température de 60 à 120°C.

22. Procédé suivant la revendication 21, dans lequel la tétralone et la 6-méthoxytétralone et le produit est le 6-méthoxy-1-cyano-3,4-dihydro-naphtalène.